# EUROPEAN PATENT APPLICATION

(11) **EP 1 611 848 A1**
(43) Date of publication of application: **04.01.2006**
(21) Application number: 05253662.0
(22) Date of filing: 14.06.2005
(51) Int. Cl.: A61B 5/15

(54) **Devices, systems and methods for extracting bodily fluid and monitoring an analyte therein**

(30) Priority: 30.06.2004 US 882994
(71) Applicant: Lifescan Scotland Ltd, Inverness IV2 3ED (GB)
(72) Inventor: Vos, Hester, Strathpfeffer IV14 9AB (GB); Kaar, Simon G., Cork (IE); Wehrheim, Frank, Pfungstadt 64319 (DE); Racchini, Joel, St Edina, Minnesota 55424 (US); Hilgers, Michael, Lake Elmo, Minnesota 55042 (US); Stout, Phil, Roseville, Minnesota 55113 (US); Rademacher, Thomas, Stillwater, Minnesota 55082 (US); Mechelke, Joel, Stillwater, Minnesota 55082 (US); Hanson, Cass A., St Paul, Minnesota 55104 (US)
(74) Representative: Tunstall, Christopher Stephen

(57) **Abstract**

Systems and methods are provided for extracting a bodily fluid sample (e.g., an interstitial fluid sample) and monitoring an analyte therein. Certain of the systems include a a penetration member (952) configured for penetrating and residing in a target site of a user's skin layer and, subsequently, extracting a fluid sample therefrom. The system also includes a pressure ring(s) (954 A-C) adapted for applying pressure to the user's skin layer in the vicinity of the target site. The system is configured such that the pressure ring(s) is capable of applying pressure in an oscillating manner. Certain methods include penetrating the skin and applying pressure to the skin in an oscillating manner.

## Description

### Background of Invention

### Field of the Invention

The present invention relates, in general, to medical devices and their associated methods and, in particular, to devices, systems and methods for extracting bodily fluid and monitoring an analyte therein.

### Description of the Related Art

In recent years, efforts in medical devices for monitoring analytes (e.g., glucose) in bodily fluids (e.g., blood and interstitial fluid) have been directed toward developing devices and methods with reduced user discomfort and/or pain, simplifying monitoring methods and developing devices and methods that allow continuous or semi-continuous monitoring. Simplification of monitoring methods enables users to self-monitor such analytes at home or in other locations without the help of health care professionals. A reduction in a user's discomfort and/or pain is particularly important in devices and methods designed for home use in order to encourage frequent and regular use. It is thought that if a blood glucose monitoring device and associated method are relatively painless, users will monitor their blood glucose levels more frequently and regularly than otherwise.

In the context of blood glucose monitoring, continuous or semi-continuous monitoring devices and methods are advantageous in that they provide enhanced insight into blood glucose concentration trends, the effect of food and medication on blood glucose concentration and a user's overall glycemic control. In practice, however, continuous and semi-continuous monitoring devices may have drawbacks. For example, during extraction of an interstitial fluid (ISF) sample from a target site (e.g., a target site in a user's skin layer), ISF flow rate may decay over time. Furthermore, after several hours of continuous ISF extraction, a user's pain and/or discomfort may increase significantly and persistent blemishes may be created at the target site.

Still needed in the field, therefore, is a device and associated method for the monitoring of an analyte (e.g., glucose) in a bodily fluid (such as ISF) that is simple to employ, creates relatively little discomfort and/or pain in a user, and facilitates continuous or semi-continuous monitoring without unduly increasing a user's pain or creating persistent blemishes. Additionally, the device needs to be relatively small and light so that it may be comfortably worn on the body for greater than about 8 hours. It is also preferable that the device operates in an automated manner which does not require frequent inputs from the user.

### SUMMARY OF INVENTION

Systems for the extraction of a bodily fluid sample and monitoring of an analyte therein according to embodiments of the present invention are simple to employ, create relatively little pain and/or discomfort in a user, and facilitate continuous and semi-continuous monitoring without unduly increasing a user's pain or creating persistent blemishes. In addition, ISF extraction devices according to embodiments of the present invention also create relatively little pain and/or discomfort in a user and facilitate continuous and semi-continuous monitoring without unduly increasing a user's pain or creating persistent blemishes. Moreover, methods according to the present invention facilitate continuous or semi-continuous monitoring without unduly increasing a user's pain or creating persistent blemishes.

Certain systems of the present invention for extracting bodily fluid include a penetration member configured for penetrating a target site in the skin and accessing bodily fluid therein, and means for applying pressure to the skin in an oscillating manner. The pressure application means includes at least one pressure ring concentrically positioned about the penetration member. The pressure application means is controlled by mechanical or electronic means to implement an oscillation frequency to the pressure ring(s). The frequency may be predetermined or may be responsive to circumstances such as fluid flow rate or volume extracted.

A system for extracting a bodily fluid sample and monitoring an analyte therein according to an exemplary embodiment of the present invention includes a disposable cartridge and a local controller module. The disposable cartridge includes a sampling module adapted to extract a bodily fluid sample (e.g., an ISF sample) from a body and an analysis module adapted to measure an analyte (for example, glucose) in the bodily fluid sample. In addition, the local controller module is in electronic communication with the disposable cartridge and is adapted to receive and store measurement data (e.g., a current signal) from the analysis module.

The sampling module of systems according to embodiments of the present invention may optionally include a penetration member configured for penetrating a target site of a user's skin layer and, subsequently, residing in the user's skin layer and extracting an ISF sample therefrom. The sampling module also optionally includes at least one pressure ring adapted for applying pressure to the user's skin layer in the vicinity of the target site while the penetration member is residing in the user's skin layer. In addition, if desired, the sampling module may be configured such that the pressure ring(s) is capable of applying pressure to the user's skin layer in an oscillating manner whereby an ISF glucose lag of the ISF sample extracted by the penetration member is mitigated.

An interstitial fluid (ISF) extraction device according to an embodiment of the present invention includes a penetration member (e.g., a thin-walled needle with a bore) configured for penetrating a target site of a user's skin layer and, subsequently, residing in a user's skin layer and extracting an ISF sample therefrom. The ISF extraction device also includes at least one pressure ring (e.g., three concentrically arranged pressure rings) adapted for applying pressure to the user's skin layer in the vicinity of the target site while the penetration member is residing in the user's skin layer. The ISF extraction device is configured such that the pressure ring(s) is capable of applying the pressure in an oscillating manner whereby an ISF glucose lag of the ISF sample extracted by the penetration member is mitigated. In addition, since the ISF extraction device is configured to apply pressure in an oscillating manner, continuous and semi-continuous monitoring is facilitated while minimizing a user's pain and the creation of persistent blemishes. Application of pressure in an oscillating manner by the pressure ring(s) may also optimize blood flow to the vicinity of the target site such that ISF glucose lag is minimized.

Since the penetration member of ISF extraction devices according to embodiments of the present invention may reside in a user's skin layer during extraction of an ISF sample, the ISF extraction devices are simple to employ.

In an embodiment of this invention, a mechanical mechanism will be described for applying pressure to the pressure rings in an automated manner. Such a device includes a gear wheel, a penetration member, a pressure ring, and a motor. The gear wheel is adapted to interact with the pressure ring such that rotation of the gear wheel in a first direction (i.e. clockwise or counter-clockwise) imparts translational motion to the pressure ring progressively towards the user's skin layer allowing pressure to be applied to the user's skin layer. In addition, rotation of the gear wheel in a second direction, opposite to the first direction, imparts translational motion to the pressure ring progressively away from the user's skin layer. In one embodiment of the invention, the pressure ring has cam mechanism, such as a helical groove which is adapted to interface with a mechanical protrusion on the gear wheel. This cam mechanism causes the rotation of the gear wheel to impart a linear motion onto the pressure ring.

Certain methods of the present invention for extracting bodily fluid from the skin, include penetrating a target site in the skin and accessing bodily fluid therein, and applying pressure to the skin in an oscillating manner substantially concentrically about the target site wherein extraction of fluid from the skin is facilitated. The pressure oscillation frequency may be predetermined or may be responsive to circumstances such as fluid flow rate or volume extracted.

A method for extracting interstitial fluid (ISF) according to an embodiment of the present invention includes providing an ISF fluid extraction device with a penetration member and at least one pressure ring. Next, a user's skin layer is contacted by the pressure ring and penetrated by the penetration member. An ISF sample is then extracted from the user's skin layer via the penetration member while applying pressure to the user's skin layer in an oscillating manner using the pressure ring(s). The oscillating manner, by which the pressure is applied, serves to mitigate an ISF glucose lag of the ISF sample extracted by the penetration member and/or to facilitate continuous or semi-continuous extraction of an ISF sample for an extended time period (e.g., an extended time period in the range of one hour to 24 hours).

These and other objects, advantages, and features of the invention will become apparent to those persons skilled in the art upon reading the details of the invention as more fully described below.

### BRIEF DESCRIPTION OF DRAWINGS

A better understanding of the features and advantages of the present invention will be obtained by reference to the following detailed description that sets forth illustrative embodiments, in which principles of the invention are utilized, and the accompanying drawings of which:

FIG. 1 is a simplified block diagram depicting a system for extracting a bodily fluid sample and monitoring an analyte therein according to an exemplary embodiment of the present invention;

FIG. 2 is a simplified schematic diagram of an ISF sampling module according to an exemplary embodiment of the present invention being applied to a user's skin layer, with the dashed arrow indicating a mechanical interaction and the solid arrows indicating ISF flow or, when associated with element 28, the application of pressure;

FIG. 3 is a simplified block diagram of an analysis module, local controller module and remote controller module according to an exemplary embodiment of the present invention;

FIG. 4 is a simplified cross-sectional side view of an extraction device according to an exemplary embodiment of the present invention;

FIG. 5 is a perspective view of a portion of an extraction device according to yet another exemplary embodiment of the present invention;

FIG. 6 is a simplified cross-sectional side view of the extraction device of FIG. 5;

FIG. 7 is a graph showing perfusion as a function of time for a test conducted using the extraction device of FIG. 4;

FIG. 8 is a flow diagram illustrating a sequence of steps in a process according to one exemplary embodiment of the present invention;

FIG. 9 is a simplified cross-sectional side view of a portion of an extraction device according a further embodiment of the present invention;

FIG. 10A is a perspective view from above of an extraction device according to another embodiment of the present invention;

FIG. 10B is a perspective view from below of the extraction device depicted in FIG. 10A;

FIG. 10C is a plan view of the extraction device depicted in FIGs. 10A-B;

FIG. 11 is a perspective exploded view of the extraction device depicted in FIGs. 10A-C;

FIG. 12 is a perspective cross-sectional view of a gear wheel of the extraction device depicted in FIG. 11;

FIG. 13 is a simplified plan view from the top of a worm wheel orthogonally interacting with the gear wheel;

FIG. 14 is a simplified cross-sectional side view of the gear wheel;

FIG. 15 is a simplified cross-sectional side view of a portion of the extraction device depicted in FIG. 11 which includes the gear wheel, an upper housing, a bottom portion of a main housing, an inner pressure ring, and outer pressure ring;

FIG. 16 shows a perspective view of the inner pressure ring.

FIG. 17 shows a perspective view of the outer pressure ring.

FIG. 18 shows a perspective cross-sectional view of the upper housing mounted to the inner pressure ring.

FIG. 19A is a cross-sectional side view of the extraction device of FIGs. 10-15 showing the position before lancing with inner pressure ring and outer pressure in their retracted state;

FIG. 19B is a cross-sectional side view of the extraction device of FIGs. 10-15 showing the position before the lancing step with the inner pressure ring in its deployed state;

FIG. 19C is a cross-sectional side view of the extraction device of FIGs. 10-15 showing the position after lancing with the inner pressure ring in its deployed state;

FIG. 19D is a cross-sectional side view of the extraction device of FIGs. 10-15 showing the position after lancing with inner pressure ring and outer pressure in their retracted state;

FIG. 19E is a cross-sectional side view of the extraction device of FIGs. 10-15 showing the position after lancing with inner pressure ring and outer pressure in their deployed state;

FIG. 20 is a simplified perspective view of an embodiment in which a glucose module attaches to an extraction device;

FIG. 21 is a simplified perspective view of a lancing module; and

FIG. 22 is a simplified cross-sectional view of the lancing module.

### Detailed Description of the Invention

Before the subject systems are described, it is to be understood that this invention is not limited to particular embodiments described or illustrated, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs.

It must be noted that as used herein and in the appended claims, the singular forms "a", "an", and "the" include plural referents unless the context clearly dictates otherwise. Thus, for example, reference to "a signal" includes a plurality of such signals and so forth.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided might be different from the actual publication dates which may need to be independently confirmed.

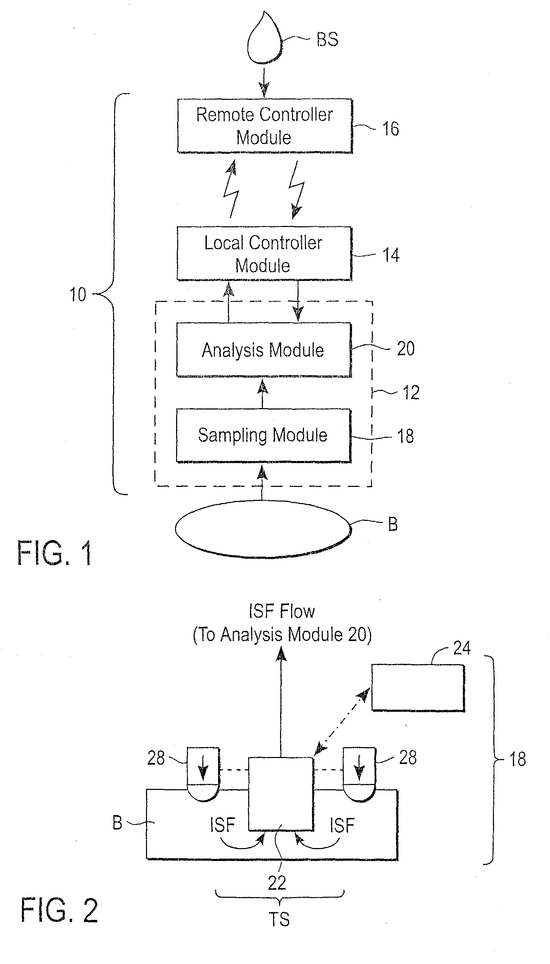

A system 10 for extracting a bodily fluid sample (e.g., an ISF sample) and monitoring an analyte (for example, glucose) therein according to an exemplary embodiment of the present invention includes a disposable cartridge 12 (encompassed within the dashed box), a local controller module 14, and a remote controller module 16, as illustrated in FIG. 1.

In system 10, disposable cartridge 12 includes a sampling module 18 for extracting the bodily fluid sample (namely, an ISF sample) from a body B, e.g., a user's skin layer, and an analysis module 20 for measuring an analyte (i.e., glucose) in the bodily fluid. Sampling module 18 and analysis module 20 may be any suitable sampling and analysis modules known to those of skill in the art. Examples of suitable sampling and analysis modules are described in International Application PCT/GB01/05634 (International Publication Number WO 02/49507 A1), which is hereby fully incorporated herein by reference. However, in system 10, sampling module 18 and analysis module 20 are both configured to be disposable since they are components of disposable cartridge 12.

As depicted in FIG. 2, the particular sampling module 18 of system 10 is, however, an ISF sampling module that includes a penetration member 22 for penetrating a target site (TS) of body B and extracting an ISF sample, a launching mechanism 24 and at least one pressure ring 28. ISF sampling module 18 is adapted to provide a continuous or semi-continuous flow of ISF to analysis module 20 for the monitoring (e.g., concentration measurement) of an analyte (such as glucose) in the ISF sample.

During use of system 10, penetration member 22 is inserted into the target site (i.e., penetrates the target site) by operation of launching mechanism 24. For the extraction of an ISF sample from a user's skin layer, penetration member 22 may be inserted to a maximum insertion depth in the range of, for example, 1.5 mm to 3 mm. In addition, penetration member 22 may be configured to optimize extraction of an ISF sample in a continuous or semi-continuous manner. In this regard, penetration member 22 may include, for example, a 25 gauge, thin-wall stainless steel needle (not shown in FIGs. 1 or 2) with a bent tip, wherein a fulcrum for the tip bend is disposed between the needle's tip and the needle's heel. Suitable needles for use in penetration members according to the present invention are described in U.S. Patent No. 6,702,791 and U.S. Patent Application Publication US 2003/0060784 A1 (Serial No. 10/185,605) which are hereby fully incorporated by reference.

Launching mechanism 24 may optionally include a hub (not shown in FIGs. 1 or 2) surrounding penetration member 22. Such a hub is configured to control the insertion depth of penetration member 22 into the target site. Insertion depth control may be beneficial during the extraction of an ISF sample by preventing inadvertent lancing of blood capillaries, which are located relatively deep in a user's skin layer, and thereby eliminating a resultant fouling of an extracted ISF sample, clogging of the penetration member or clogging of an analysis module by blood. Controlling insertion depth may also serve to minimize pain and/or discomfort experienced by a user during use of system 10.

Although FIG. 2 depicts launching mechanism 24 as being included in sampling module 18, launching mechanism 24 may optionally be included in disposable cartridge 12 or in local controller module 14 of system 10. Furthermore, to simplify employment of system 10 by a user, sampling module 18 may be formed as an integral part of the analysis module 20.

In order to facilitate the extraction of a bodily fluid (e.g., ISF) from the target site, penetration member 22 may be arranged concentrically within at least one pressure ring 28. Pressure ring(s) 28 may be of any suitable shape, including but not limited to, annular. An example of such an arrangement is disclosed in U.S. Patent No. 5,879,367 which is hereby fully incorporated by reference.

During use of system 10, pressure ring 28 is applied in the vicinity of the target site TS, prior to penetration of the target site by penetration member 22, in order to tension the user's skin layer. Such tension serves to stabilize the user's skin layer and to prevent tenting thereof during penetration by the penetrating member. Alternatively, stabilization of the user's skin layer prior to penetration by the penetrating member may be achieved by a penetration depth control element (not shown) included in sampling module 18. Such a penetration depth control element rests or "floats" on the surface of the user's skin layer, and acts as a limiter for controlling penetration depth (also referred to as insertion depth). Examples of penetration depth control elements and their use are described in U.S. Patent Application Serial No. 10/690,083, which is hereby fully incorporated herein by reference.

Once penetration member 22 has been launched and has penetrated the target site TS, a needle (not shown in FIGs. 1 or 2) of penetration member 22 will reside, for example, at an insertion depth in the range of about 1.5 mm to 3 mm below the surface of the user's skin layer. If desired, penetration member 22 may be launched coincidentally with application of pressure ring(s) 28 to the user's skin layer, thereby enabling a simplification of the launching mechanism. The pressure ring(s) 28 applies/apply a force on the user's skin layer (indicated by the downward pointing arrows of FIG. 2) that pressurizes ISF in the vicinity of the target site. A sub-dermal pressure gradient induced by the pressure ring(s) 28 results/result in flow of ISF up the needle and through the sampling module to the analysis module (as indicated by the curved and upward pointing arrows of FIG. 2).

ISF flow through a penetration member's needle is subject to potential decay over time due to depletion of ISF near the target site and due to relaxation of the user's skin layer under the pressure ring(s) 28. The systems and methods of the present invention address this by varying one or more aspects of the applied pressure.

In one variation, the amount of applied pressure may be varied over a given time. While contact between the pressure ring(s) and the skin might be constant, the amount of that pressure may be varied. For example, the amount of pressure may be progressively increased proportionately or otherwise to the volume or flow rate of the ISF being extracted. Alternatively, the increase in pressure may be staggered or applied in a stepwise fashion. Still yet, the pressure may be oscillated between various levels of greater and lesser pressure, where the reduction in pressure may include the discontinuance of pressure by completely removing the pressure ring(s) from contact with the skin. The oscillation frequency of the pressure ring(s) may be constant or varied depending on the application. For example, the application times of higher pressure ("on") and lower or no pressure ("off") may be the same (e.g., 3 minutes on followed by 3 minutes off, etc.) or different (e.g., 15 minutes on followed by 10 minutes off, etc.) or one may be constant and the other may vary (e.g., 15 minutes on followed by 20 minutes off followed by 15 minutes on followed by 10 minutes off, etc.).

In other variations of the invention, while the amount of applied pressure to the skin may be constant over a period of time, the location of that pressure relative to the needle penetration site may vary over time. For example, the initial pressure may commence at a certain radial distance (assuming a substantially annular configuration of the pressure ring) from the penetration site where that radial distance is reduced or increased over time. The change in distance may be gradual or less so depending on the application or in response to ISF extraction flow or volume. This may be accomplished by the use of multiple pressure rings having varying diameters which are individually and successively applied to the target site.

Still yet, the location of the initial pressure may be maintained, but the radial surface area over which the pressure is applied may be increased or decreased. In other words, the amount of surface area of the pressure ring in contact with the skin may be increased or increased. This may also be accomplished by the use of multiple pressure rings which are individually but cumulatively applied or successively removed from application to the skin.

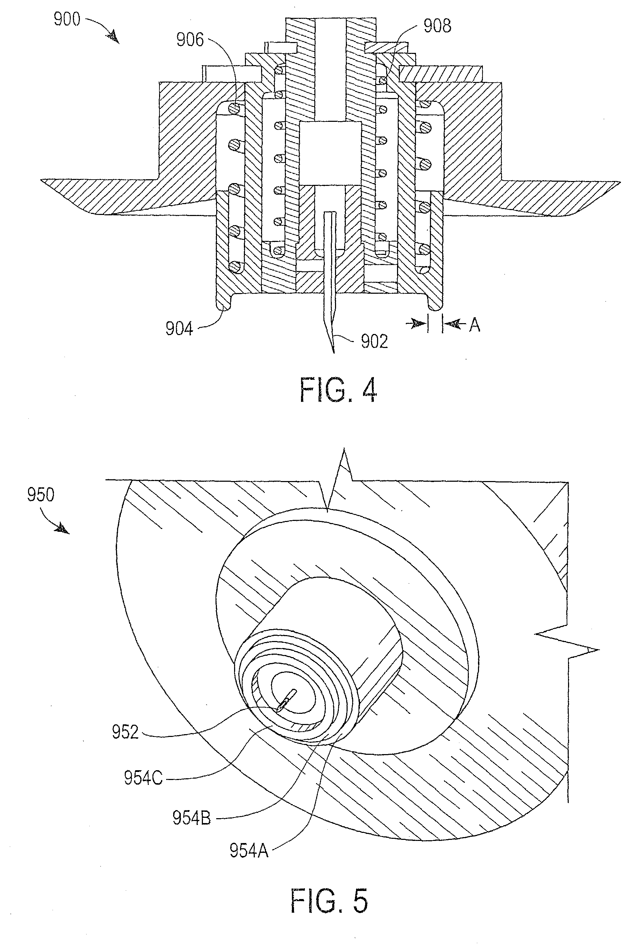

Returning to the figures, and as mentioned above, pressure ring(s) 28 may be applied to the user's skin layer in an oscillating manner (e.g., with a predetermined pressure ring(s) cycling routine or with a pressure ring cycling routine that is controlled via and is responsive to ISF flow rate measurement and feedback) while the penetration member is residing in the user's skin layer in order to minimize ISF flow decay. In addition, during application of pressure in an oscillating manner, there may be time periods during which the pressure applied by the pressure ring(s) is varied or the local pressure gradient is removed and the net outflow of ISF from the user's skin layer is eliminated. In addition, pressure ring(s) 28 may be configured to apply an oscillating mechanical force (i.e., pressure) in the vicinity of the target site while the penetration member is residing in the user's skin layer. Such oscillation may be achieved through the use of a biasing element (not shown in FIGs. 1 or 2), such as a spring or a retention block. The structure and function of a pressure ring(s) in sampling modules (and ISF extraction devices) according to the present invention are described in more detail below with respect to FIGs. 4-7 and 10-19.

Furthermore, alternating the application of a plurality of pressure rings to the user's skin layer in the vicinity of the target site may serve to control the flow of ISF through the sampling and analysis modules and limit the time that any given portion of the user's skin layer is under pressure. By allowing a user's skin layer to recover, the application of pressure in an oscillating manner also reduces blemishes on the user's skin and a user's pain and/or discomfort. An additional beneficial effect of applying pressure ring(s) 28 in an oscillating manner is that ISF glucose lag (i.e., the difference between glucose concentration in a user's ISF and glucose concentration in a user's blood) is reduced.

Once apprised of the present disclosure, one skilled in the art may devise a variety of pressure ring cycling routines that serve to reduce ISF glucose lag, a user's pain/discomfort and/or the creation of persistent skin blemishes. For example, the pressure ring(s) 28 may be deployed (i.e., positioned such that pressure is applied to a user's skin layer in the vicinity of a target site) for a period of from 30 seconds to 3 hours and may then be retracted (i.e., positioned such that pressure is not being applied to the user's skin layer) for a period ranging from 30 seconds to 3 hours. Moreover, it has been determined that ISF glucose lag and a user's pain/discomfort are significantly reduced when the amount of time during which pressure is applied (i.e., the time period during which at least one pressure ring is deployed) is in the range of about 30 seconds to about 10 minutes and the amount of time during which pressure is released (i.e., the time period during which the at least one pressure ring is retracted) is in the range of about 5 minutes to 10 minutes. A particularly beneficial pressure ring cycle includes the application of pressure for one minute and the release of pressure for 10 minutes. Since different amounts of time are used for applying and releasing pressure, such a cycle is referred to as an asymmetric pressure ring cycle.

Pressure ring cycling routines may be devised such that the following concerns are balanced: (i) having the pressure ring(s) deployed for a time period that is sufficient to extract a desired volume of bodily fluid, (ii) inducing a physiological response that mitigates ISF glucose lag, and (iii) minimizing user discomfort and the creation of persistent blemishes. In addition, pressure ring cycling routines may also be devised to provide for semi-continuous analyte measurements that occur, for example, every 15 minutes.

Pressure ring(s) 28 may be formed of any suitable material known to those of skill in the art. For example, the pressure ring(s) 28 may be composed of a relatively rigid material, including, but not limited to, acrylonitrile butadiene styrene plastic material, injection moldable plastic material, polystyrene material, metal or combinations thereof. The pressure ring(s) 28 may also be composed of relatively resiliently deformable material, including, but not limited to, elastomeric materials, polymeric materials, polyurethane materials, latex materials, silicone materials or combinations thereof.

An interior opening defined by the pressure ring(s) 28 may be in any suitable shape, including but not limited to circular, square, triangular, C-shape, U-shape, hexagonal, octagonal and crenellated shape.

When pressure ring(s) 28 is being employed to minimize ISF flow decay and/or control the flow of ISF through the sampling and analysis modules, penetration member 22 remains deployed in (i.e., residing in) the target site of the user's skin layer while the pressure ring(s) 28 is/are in use. However, when pressure ring(s) 28 are being employed to mitigate ISF glucose lag, the penetration member 22 may intermittently reside in the user's skin layer. Such intermittent residence of the penetration member 22 may occur either in or out of concert with the application of pressure by the pressure ring(s) 28.

Any suitable glucose sensor known to those of skill in the art may be employed in analysis modules according to the present invention. Glucose sensor 310 may contain, for example, a redox reagent system including an enzyme and a redox active compound(s) or mediator(s). A variety of different mediators are known in the art, such as ferricyanide, phenazine ethosulphate, phenazine methosulfate, pheylenediamine, 1-methoxy-phenazine methosulfate, 2,6-dimethyl-1,4-benzoquinone, 2,5-dichloro-1,4-benzoquinone, ferrocene derivatives, osmium bipyridyl complexes, and ruthenium complexes. Suitable enzymes for the assay of glucose in whole blood include, but are not limited to, glucose oxidase and dehydrogenase (both NAD and PQQ based). Other substances that may be present in the redox reagent system include buffering agents (e.g., citraconate, citrate, malic, maleic, and phosphate buffers); divalent cations (e.g., calcium chloride, and magnesium chloride); surfactants (e.g., Triton, Macol, Tetronic, Silwet, Zonyl, and Pluronic); and stabilizing agents (e.g., albumin, sucrose, trehalose, mannitol and lactose).

In an embodiment in which the analysis module includes an electro-chemical based glucose sensor, the glucose sensor may produce an electrical current signal in response to the presence of glucose in an ISF sample. Local controller module 14 may then receive the electrical current signal via electrical contacts (not shown) and converts that signal to one that is representative of the ISF glucose concentration.

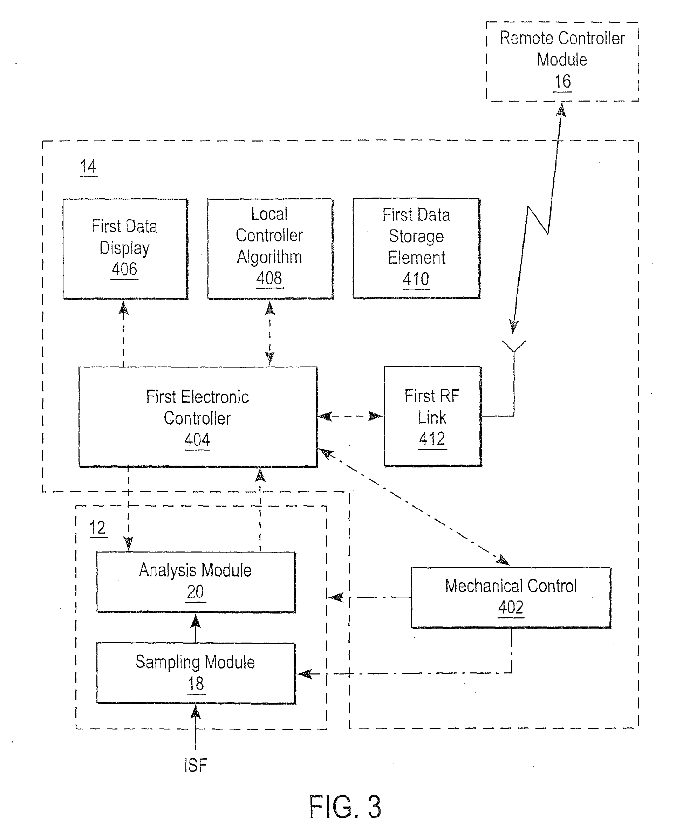

Local controller module 14 is depicted in simplified block form in FIG. 3. Local controller module 14 includes a mechanical controller 402, a first electronic controller 404, a first data display 406, a local controller algorithm 408, a first data storage element 410 and a first RF link 412. Local controller module 14 is configured such that it may be electrically and mechanically coupled to disposable cartridge 12. The mechanical coupling provides for disposable cartridge 12 to be removably attached to (e.g., inserted into) local controller module 14. Local controller module 14 and disposable cartridge 12 are configured such that they may be attached to the skin of a user by, for example, by a strap, in a manner which secures the combination of the disposable cartridge 12 and local controller module 14 onto the user's skin.

During use of system 10, first electronic controller 404 controls the measurement cycle of the analysis module 20, as described above. Communication between local controller module 14 and disposable cartridge 12 takes place via the electrical contacts on analysis module 20 and the corresponding electrical contacts on local controller module 14. Electrical signals representing the glucose concentration of an ISF sample are then sent by the analysis module to the local controller module. First electronic controller 404 interprets these signals by using the local controller algorithm 408 and displays measurement data on a first data display 406 (which is readable by the user). In addition, measurement data (e.g., ISF glucose concentration data) may be stored in first data storage element 410.

Prior to use, an unused disposable cartridge 12 is inserted into local controller module 14. This insertion provides for electrical communication between disposable cartridge 12 and local controller module 14. A mechanical controller 402 in the local controller module 14 securely holds the disposable cartridge 12 in place during use of system 10.

After attachment of a local controller module and disposable cartridge combination to the skin of the user, and upon receiving an activation signal from the user, a measurement cycle is initiated by first electronic controller 404. Upon such initiation, penetration member 22 is launched into the user's skin layer to start ISF sampling. The launching may be initiated either by first electronic controller 404 or by mechanical interaction by the user.

First RF link 412 of local controller module 14 is configured to provide bidirectional communication between the local controller module and a remote controller module 16, as depicted by the jagged arrows of FIGs. 1 and 3. The local controller module incorporates a visual indicator (e.g., a multicolor LED) indicating the current status, e.g., a red light may be used to indicate a hypo or hyperglycemic state and a green light may be used to indicate a euglycemic state, etc., of the system.

Local controller module 14 is configured to receive and store measurement data from, and to interactively communicate with, disposable cartridge 12. For example, local controller module 14 may be configured to convert a measurement signal from analysis module 20 into an ISF or blood glucose concentration value.

The difference between an ISF glucose value (concentration) at any given moment in time and a blood glucose value (concentration) at the same moment in time is referred to as the ISF glucose lag. ISF glucose lag may be conceivably attributed to both physiological and mechanical sources. The physiological source of lag in ISF glucose is related to the time it takes for glucose to diffuse between the blood and interstices of a user's skin layer. The mechanical source of lag is related to the method and device used to obtain an ISF sample.

Embodiments of devices, systems and methods according to the present invention mitigate (reduce or minimize) ISF glucose lag due to physiological sources by applying and releasing pressure to a user's skin layer in an oscillating manner that enhances blood flow to a target area of the user's skin layer. ISF extraction devices that include pressure ring(s) according to the present invention (as described in detail below) apply and release pressure in this manner. Another approach to account for lag in ISF glucose is to employ an algorithm (e.g., predictive algorithm) that predicts blood glucose concentration based on measured ISF glucose concentrations.

Predictive algorithm may, for example, take the general form:
Predicted blood glucose = f(ISFᵢ^{k}, rateⱼ, maₙrateₘ^{p}, interaction terms)
where:
i is an integer of value between 0 and 3;
j, n, and m are integers of value between 1 and 3;
k and p are integers of value 1 or 2;
ISFi is a measured ISF glucose value with the subscript (i) indicating which ISF value is being referred to, i.e., 0 = current value, 1 = one value back, 2 = two values back, etc.;
ratej is the rate of change between adjacent ISF values with the subscript (i) referring to which adjacent ISF values are used to calculate the rate, i.e., 1 = rate between current ISF value and the previous ISF value, 2= rate between the ISF values one previous and two previous relative to the current ISF value, etc.; and
manratem is the moving average rate between adjacent averages of groupings of ISF values, with the subscripts (n) and (m) referring to (n) the number of ISF values included in the moving average and (m) the time position of the moving adjacent average values relative to the current values as follows.

The general form of the predictive algorithm is a linear combination of all allowed terms and possible cross terms, with coefficients for the terms and cross terms determined through regression analysis of measured ISF values and blood glucose values at the time of the ISF sample acquisition. Further details regarding predictive algorithms suitable for use in systems according to the present invention are included in U.S. Patent Application Serial No. 10/652,464 filed on August 28, 2003, which is hereby incorporated by reference.

As will also be appreciated by those skilled in the art, the outcome of the predictive algorithm may be used to control medical devices such as insulin delivery pumps. A typical example of a parameter that may be determined based on the algorithm outcome is the volume of a bolus of insulin to be administered to a user at a particular point in time.

FIG. 4 is a cross-sectional side view of an interstitial fluid (ISF) extraction device 900 according to an exemplary embodiment of the present invention. ISF extraction device 900 includes a penetration member 902, a pressure ring 904, a first biasing member 906 (i.e., a first spring) and a second biasing member 908 (namely, a second spring).

Penetration member 902 is configured for penetration of a user's skin layer at a target site and for the subsequent extraction of ISF therefrom. Penetration member 902 is also configured to remain in (reside in) the user's skin layer during the extraction of ISF therefrom. Penetration member 902 can, for example, remain in the user's skin layer for more than one hour, thus allowing a continuous or semi-continuous extraction of ISF. Once apprised of the present disclosure, one skilled in the art will recognize that the penetration member may reside in the user's skin layer for an extended period of time of 8 hours or more.

Pressure ring 904 is configured to oscillate between a deployed state and a retracted state. When pressure ring 904 is in the deployed state, it applies pressure to the user's skin layer surrounding the target site, while the penetration member is residing in the user's skin layer in order to (i) facilitate the extraction of ISF from the user's skin layer and (ii) control the flow of ISF through ISF extraction device 900 to, for example, an analysis module as described above. When pressure ring 904 is in a retracted state, it applies either a minimal pressure or no pressure to the user's skin layer surrounding the target site. Since pressure ring 904 may be oscillated between a deployed state and a retracted state, the time that any given portion of a user's skin layer is under pressure may be controlled, thereby providing for the user's skin layer to recover and reducing pain and blemishes.

Pressure ring 904 typically has, for example, an outside diameter in the range from about 0.08 inches to about 0.56 inches and a wall thickness (depicted as dimension "A" in FIG. 4) in the range from about 0.02 inches to about 0.04 inches.

First biasing element 906 is configured to urge pressure ring 904 against the user's skin layer (i.e., to place pressure ring 904 into a deployed state) and to retract pressure ring 904. Second biasing element 908 is configured to launch the penetration member 902 such that the penetration member penetrates the target site.

The pressure (force) applied against a user's skin layer by the pressure ring(s) may be, for example, in the range of from about 1 to about 150 pounds per square inch (PSI, calculated as force per cross-sectional pressure ring area), and more typically in the range from about 30 to about 70 PSI. In this regard, a pressure of approximately 50 PSI has been determined to be beneficial with respect to providing adequate ISF flow while minimizing user pain/discomfort.

In the embodiment of FIG. 4, penetration member 902 is partially housed in a recess of the oscillating pressure ring 904, the depth of the recess determining the maximum penetration depth of the penetration member 902. Although not explicitly shown in FIG. 4, the penetration member 902 and the oscillating pressure ring 904 may be moved relative to one another and applied to a user's skin layer independent of each other.

During use of ISF extraction device 900, the oscillating pressure ring 904 may be deployed for stabilizing the user's skin layer and to isolate and pressurize a region of the target area and thus to provide a net positive pressure to promote flow of ISF through penetration member 902.

If desired, ISF extraction device 900 may contain a penetration depth control element (not shown) for limiting and controlling the depth of needle penetration during lancing. Examples of suitable penetration depth control elements and their use are described in U.S. patent application Serial No. 10/690,083 [Attorney Docket No. LFS-5002], which is hereby fully incorporated herein by reference.

During use of ISF extraction device 900, a system that includes ISF extraction device 900 is placed against a user's skin layer with the pressure ring 904 facing the skin (see, for example, FIG. 4). The pressure ring 904 is urged against the skin to create a bulge. The bulge is then penetrated (e.g., lanced) by the penetration member 902. An ISF sample is subsequently extracted from the bulge while the penetration member 902 remains totally or partially within the skin.

The flow rate of the ISF sample being extracted is initially relatively high but typically declines over time. After a period in the range of 3 seconds to 3 hours, pressure ring 904 may be retracted to allow the skin to recover for a period of about 3 seconds to 3 hours. Pressure ring 904 may then be re-deployed for a period in the range of about 3 seconds to about 3 hours and retracted for about 3 seconds to 3 hours. This process of deploying and retracting pressure ring 904 proceeds until ISF extraction is discontinued. The deployment and retraction cycles are preferably asymmetric in that different periods of time are used for each cycle, e.g., the deployment cycle may be different from the retraction cycle, or the deployment (or retraction) cycles are different from each other in each successive cycle, or both.

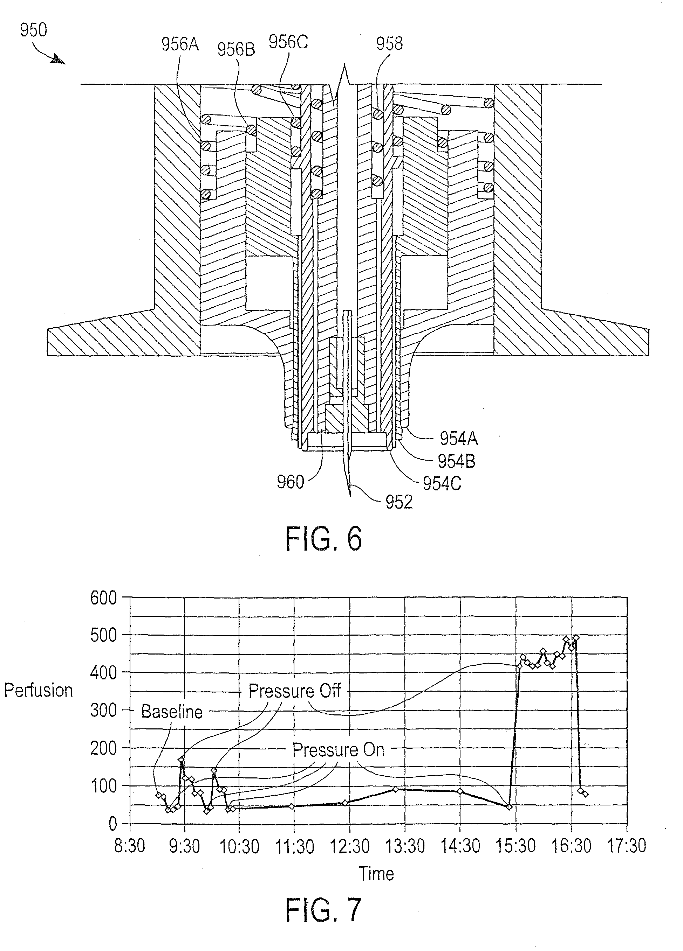

FIGs. 5 and 6 are cross sectional and perspective views, respectively, of an ISF extraction device 950 according to another exemplary embodiment of the present invention. ISF extraction device 950 includes a penetration member 952 and a plurality of concentrically arranged pressure rings 954A, 954B and 954C. ISF extraction device 950 also includes a plurality of first biasing elements 956A, 956B and 956C for urging the pressure rings 954A, 954B and 956C, respectively, toward and against a user's skin layer, a second biasing element 958 for launching the penetration member 952, and a penetration depth control element 960.

During use, ISF extraction device 950 is positioned such that pressure rings 954A, 954B and 954C are facing a user's skin layer. This may be accomplished, for example, by employing ISF extraction device 950 in a sampling module of a system for extracting bodily fluid as described above and placing the system against the user's skin layer.

Pressure ring 954A is then urged against the user's skin layer by biasing element 956A, thereby creating a bulge in the user's skin layer that will subsequently be lanced (i.e., penetrated) by penetration member 952. While pressure ring 954A is in use (i.e., deployed), pressure ring 954B and pressure ring 954C may be maintained in a retracted position by biasing elements 956B and 956C, respectively.

ISF may be extracted from the bulge formed in user's skin layer while the penetration member 952 resides totally or partially within the user's skin layer. After about 3 seconds to 3 hours, the pressure ring 954A may be retracted to allow the user's skin layer to recover for a time period in the range of about 3 seconds to 3 hours. After retracting the pressure ring 954A, pressure ring 954B may be deployed to apply pressure on the user's skin layer. While pressure ring 954B is in use (i.e., deployed), pressure ling 954A and pressure ring 954C may be maintained in a retracted position by biasing elements 956A and 956C, respectively. After a time period of about 3 seconds to 3 hours, pressure ring 954B may be retracted for a time period in the range of 3 seconds to 3 hours, followed by the deployment of pressure ring 954C. Pressure ring 954C maintains pressure on the user's skin layer for a time period in the range of 3 seconds to 3 hours and is then retracted for a time period in the range of 3 seconds to 3 hours. While pressure ring 954C is in use (i.e., deployed), pressure ring 954A and pressure ring 954B may be maintained in a retracted position by biasing elements 956A and 956B, respectively. This process of cycling between deployment and retraction of pressure rings 954A, 954B and 954C may proceeds until fluid extraction has ended. As with the embodiment shown in FIG. 4, the deployment and retraction cycles in the multiple pressure ring embodiment of FIGs. 5 and 6 are preferably asymmetric in that different periods of time are used for each cycle.

Those skilled in the art will also recognize that a plurality of pressure rings in ISF extraction devices according to the present invention may be deployed in any order and that one is not limited to the deployment and retraction sequence described above. For example, a sequence may be used in which pressure ring 954B or 954C is applied before pressure ring 954A. Further, more than one pressure ring may be deployed simultaneously. For example, the embodiment shown in FIGs. 5 and 6 may deploy all three pressure rings simultaneously such that the pressure rings function as a single pressure ring.

For the embodiment shown in FIGs. 5 and 6, the pressure applied against the user's skin can, for example, range from about 0.1 to about 150 pounds per square inch (PSI) for each of the plurality of pressure rings.

The pressure rings 954A, 954B and 954C may have, for example, outer diameters of in the range of 0.08 to 0.560 inches, 0.1 to 0.9 inches and 0.16 to 0.96 inches, respectively. The wall thickness of each pressure ring may be, for example, in the range of 0.02 to 0.04 inches.

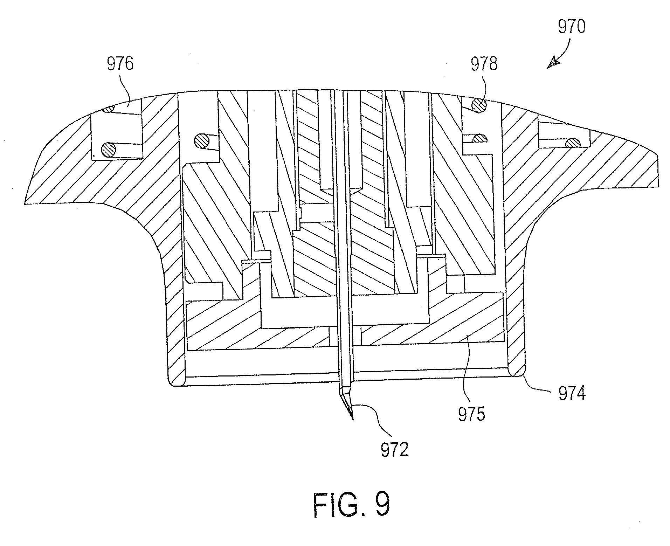

An inner-most pressure ring of extraction devices according to an alternative embodiment of the present invention can, if desired, be a flat ring (see FIG. 9 for the purpose of keeping the needle in the user's skin layer while applying negligible pressure to keep blood flowing to the area. FIG. 9 shows a cross-sectional side view of a portion of an interstitial fluid (ISF) extraction device 970 according to an alternative exemplary embodiment of the present invention. ISF extraction device 970 includes a penetration member 972, a pressure ring 974, a flat pressure ring 975, a first biasing member 976 (i.e., a first spring) for biasing the pressure ring 974 and a second biasing member 978 (namely, a second spring) for biasing the flat pressure ring.

In this alternate embodiment, the flat pressure ring surrounds the needle (i.e., the penetration member 972) and contains a hole of sufficient size to just allow the needle to pass through. The flat pressure ring preferably has a diameter of 0.02 to 0.56 inches.

ISF extraction device 900, which uses a spring for biasing element 906, is not easily adapted for automated use such that the pressure ring may oscillate several times between the deployed and retracted state. A motor would be required to pre-tension the springs for ISF extraction device 900. However, if a motor was integrated into ISF extraction device 900, then a spring would not be necessary because the motor could be used to directly apply a force to the pressure ring(s). In an improved embodiment of ISF extraction device 900, ISF extraction device 1500 was constructed using two concentric pressure rings and a motor to enable an automated oscillation of the pressure rings between the deployed and retracted state.

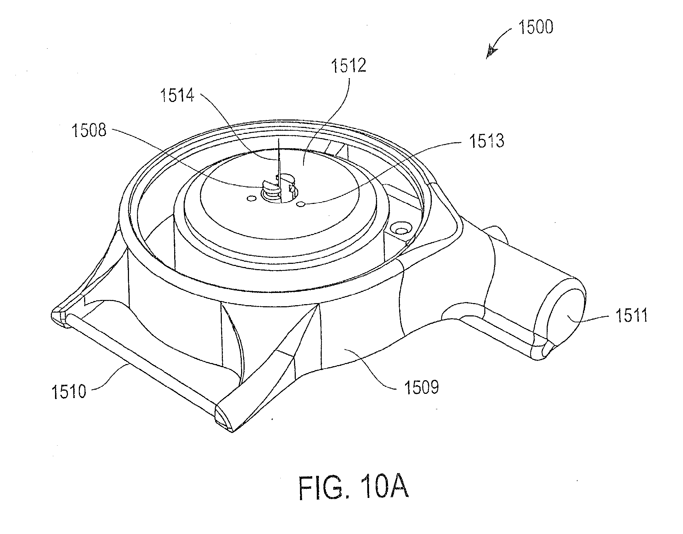

FIGs. 10A-C show a perspective top, a perspective bottom, and a top plan view, respectively, of ISF extraction device 1500 which includes a main housing 1509, two strap handles 1510, a worm wheel port 1511, a worm wheel 1528, an upper housing 1512, a lance positioner hole 1513, a penetration member 1514, inner pressure ring 1517, outer pressure ring 1518, a hole 1519, and a tab 1520.

It is preferable that ISF extraction device 1500 be relatively small and light so that it may be comfortable to a user when wearing ISF extraction device 1500 for about 8 hours or greater. A strap (not shown) may be attached to strap handles 1510 allowing ISF extraction device 1500 to be attached to the body in a manner similar to a wristwatch. In addition, ISF extraction device 1500 may be attached to a forearm, an upper arm, or an abdomen. In an embodiment of this invention, ISF extraction device 1500 has a height of about 14 mm and a diameter of about 45 mm. In addition, ISF extraction device 1500 may have a weight of about 30 grams to about 50 grams. In the process of using ISF extraction device 1500, a bottom portion 1516 of ISF extraction device 1500 may be mounted onto the skin. In one embodiment of this invention, a double-sided pressure sensitive adhesive may be deposed on bottom portion 1516, in lieu of the wrist strap, to immobilize ISF extraction device 1500. In another embodiment of this invention, the double-sided pressure sensitive adhesive may be deposed on bottom portion 1516, in conjunction with the wrist strap, to further immobilize extraction device 1500.

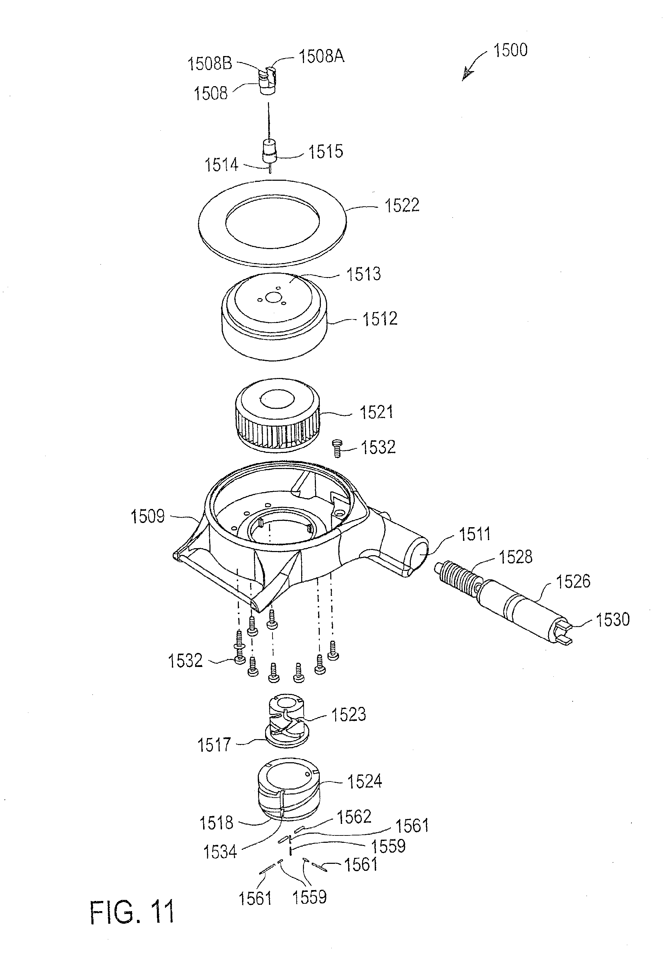

It is preferable that inner pressure ring 1517 and outer pressure ring 1518 move towards the skin and away from the skin in an automated manner. First electronic controller 404 may be used to manage the movement of inner pressure ring 1517 and outer pressure ring 1518 by controlling the duration and direction of a motor 1526. Inner pressure ring 1517 and/or outer pressure ring 1518, in their deployed state, may extend downward up to about 8 mm towards the user's skin layer causing a pressure to be generated thereto. If a spring was used to apply a comparable amount of pressure required to extend up to about 8 mm towards the user's skin layer, the height of an ISF extraction device would become relatively large. In an embodiment of this invention, the motor may enable inner pressure ring 1517 or outer pressure ring 1518 to apply a pressure (force) of about 0.1 PSI to about 150 PSI. In order to decrease the height while providing a sufficient amount of force, ISF extraction device 1500 uses a cam mechanism integrated with a gear wheel which will be described in FIGs. 11-19.

FIG. 11 is a perspective exploded view of ISF extraction device 1500which further includes a clamp 1508, a penetration member bracket 1515, a cover 1522, a gear wheel 1521, a screw 1532, a motor 1526, a worm wheel 1528, power contacts 1530, a fixing pin 1562, first surface pin 1559, and second surface pin 1561. Inner pressure ring 1517 and outer pressure ring 1518 have a respective first profile 1523 and second profile 1524 as shown in FIG. 11. Both first profile 1523 and second profile 1524 are helically shaped and recessed (i.e., eccentrically or spirally grooved). Motor 1526 is used to rotate worm wheel 1528 which is located within port 1511 and secured in place by screw 1532. In an embodiment of this invention, motor 1526 may be a rotary DC motor or a stepper motor. Power contacts 1530 may be electrically attached to a battery or a power supply to allow power to be sent to motor 1526.

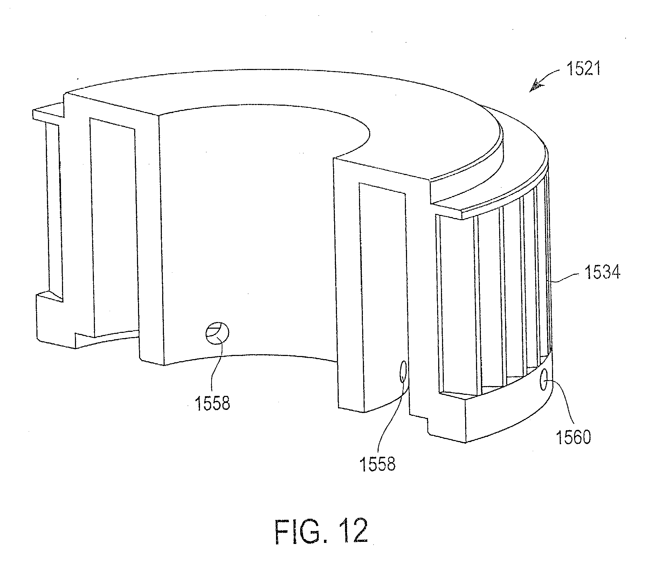

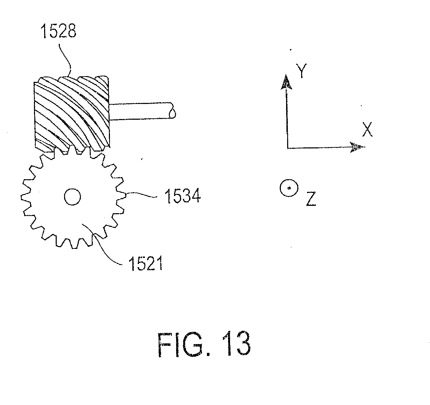

Gear wheel 1521 has a plurality of teeth 1534 on its periphery as shown in FIG. 12. More specifically, gear wheel 1521 as shown in FIG. 11 may also be referred to as a worm gear which is adapted to mechanically interact with worm wheel 1528. Teeth 1534 may orthoganally interact with worm wheel 1528 allowing gear wheel 1521 to rotate as shown in FIG. 13. Worm wheel 1528 may rotate in a clockwise or counterclockwise direction around the X-axis which in turn causes gear wheel 1521 to rotate in a clockwise or counterclockwise direction around the Z-axis. In one embodiment of this invention, motor 1526 is situated on a side of main housing 1509 as opposed to on top of upper housing 1512 thereby allowing ISF extraction device 1500 to have a relatively small height. Motor 1526 causes worm wheel 1528 to rotate, which in turn, causes gear wheel 1521 to rotate which imparts a translational motion to inner pressure ring 1517 and/or outer pressure ring 1518. The translational motion may move either inner pressure ring 1517 or outer pressure ring 1518 to their deployed or retracted state.

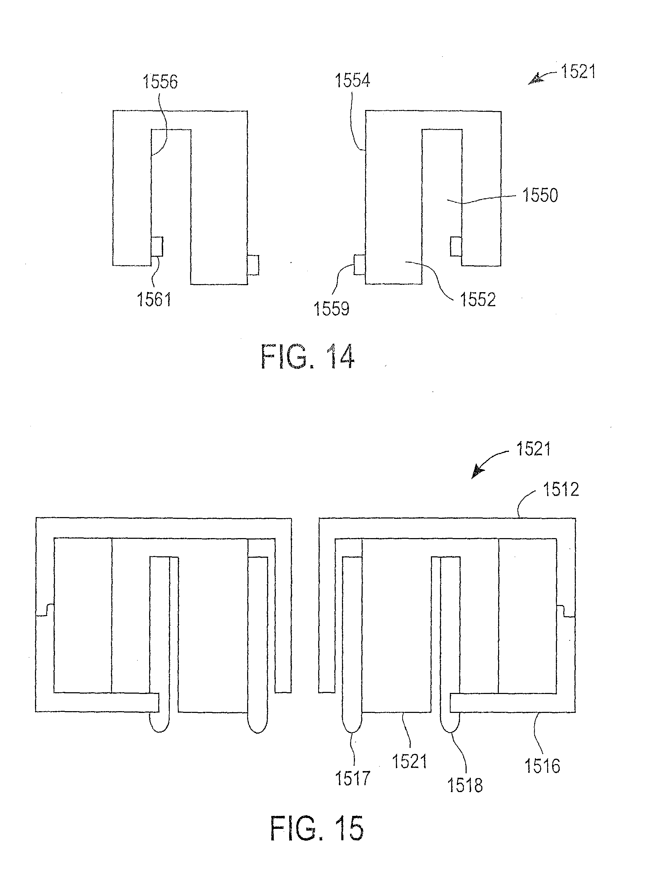

In an embodiment of this invention, gear wheel 1521 includes an annular space 1550 formed by a concentric cylinder 1552 inside of gear wheel 1521 as shown in FIGs. 14-15. Gear wheel 1521 further includes a first surface 1554 having a first surface hole 1558 and a second surface 1556 having a second surface hole 1560.

First profile pin 1559 (see FIG. 11) may be fixedly mounted to first surface hole 1558 (see FIG. 12). First profile pin 1559 may then be keyed to interface with a first profile 1523 which is situated on an outermost portion of inner pressure ring 1517. In an embodiment of this invention, there may be three first profile pins 1559 which are used to impart translational motion to inner pressure ring 1517. It should be obvious to one skilled in the art that the function of first profile pin 1559 could also be implemented by constructing a mechanical protrusion on first surface 1554 or on the outermost surface of inner pressure ring 1517. For the situation in which the mechanical protrusion is situated on the outermost surface of inner pressure ring 1517, first profile 1523 must then be situated on first surface 1554.

Similarly, second profile pin 1561 (see FIG. 11) may be fixedly mounted to second surface hole 1560 (see FIG. 12). Second profile pin 1561 may then be keyed to interface with a second profile 1524 which is situated on an outermost portion of outer pressure ring 1518. In an embodiment of this invention, there may be three second profile pins 1561 which are used to impart translational motion to outer pressure ring 1518. It should be obvious to one skilled in the art that the function of second profile pin 1561 could also be implemented by constructing a mechanical protrusion on second surface 1556 or on the outermost surface of outer pressure ring 1518. For the situation in which the mechanical protrusion is situated on the outermost surface of outer pressure ring 1518, second profile 1524 must then be situated on second surface 1556.

Inner pressure ring 1517 may be slidingly adapted to first surface 1554 as shown in FIG. 15. In addition, outer pressure ring 1518 may be slidingly adapted to fit within annular space 1550.. An upper housing and a bottom portion 1516 of main housing 1509 form a sandwich structure to secure gear wheel 1521, inner pressure ring 1517, and outer pressure ring 1518. The sandwich structure is held together by a plurality of screws 1532 as shown in FIG. 11.

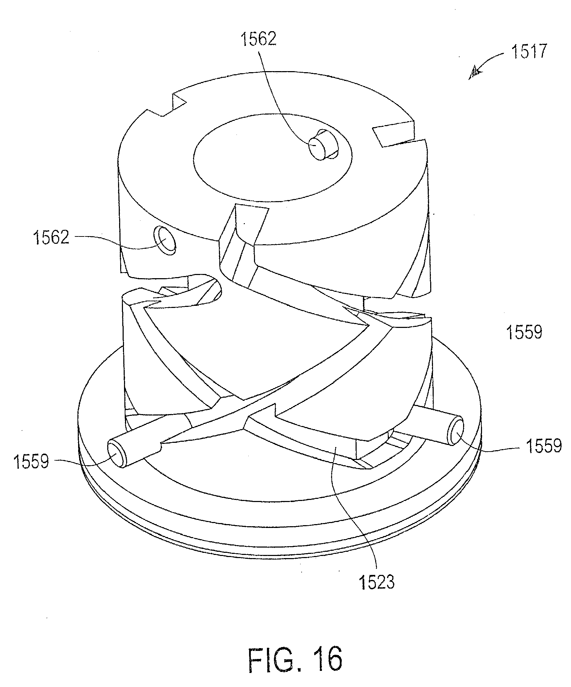

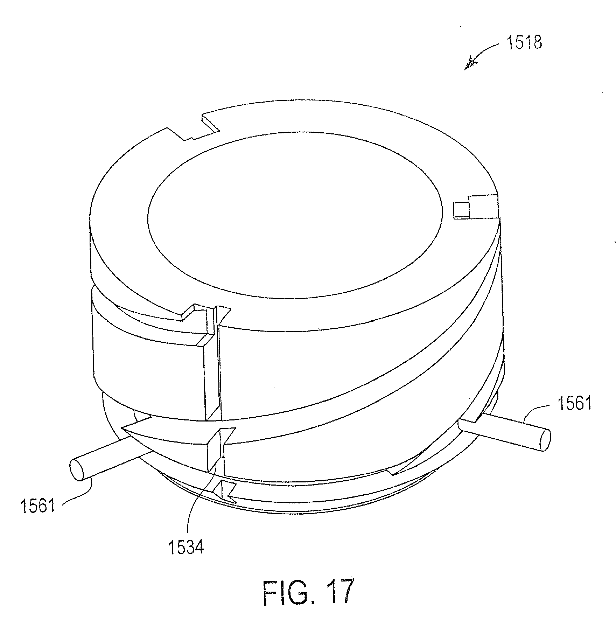

Both inner pressure ring 1517 and outer pressure ring have a cam mechanism which may convert a rotational motion of gear wheel 1521 to a linear reciprocating motion. FIG. 16 shows a perspective view of inner pressure ring 1517. In an embodiment of this invention, first profile 1523 may have a criss-crossed helical groove allowing inner pressure ring 1517 to move downward, in a linear manner, as a result of either a clockwise or counterclockwise motion of gear wheel 1521. First surface pin 1559 is adapted to interact with first profile 1523 allowing the rotational motion of gear wheel 1521 to be translated into a linear motion of inner pressure ring 1517. FIG. 17 shows a perspective view of outer pressure ring 1518. In an embodiment of this invention, second profile 1524 may have a single helical groove allowing outer pressure ring 1518 to move downward, in a linear manner, as a result of a rotational motion of gear wheel 1521. Second surface pin 1561 is adapted to interact with second profile 1524 allowing the rotational motion of gear wheel 1521 to be translated into a linear motion of outer pressure ring 1518. First profile 1523 may have a pitch of about two times greater than second profile 1524 causing inner pressure ring 1517 to have a linear displacement motion which is about 2 times greater than outer pressure ring 1518 per unit revolution of gear wheel 1521. For example, a continuous clockwise motion of gear wheel 1521 may cause inner pressure ring 1517 to become deployed first followed by the subsequent deployment of outer pressure ring 1518. A continuous counter clockwise motion of gear wheel 1521 may cause only inner pressure ring 1517 to become deployed. It should be obvious to one skilled in the art that numerous sequences of pressure ring motion may be implemented by changing a pattern of either first profile 1523 and/or second profile 1524. Additionally, the direction and duration of motor 1526 may be modified to control the sequence of pressure ring motion.

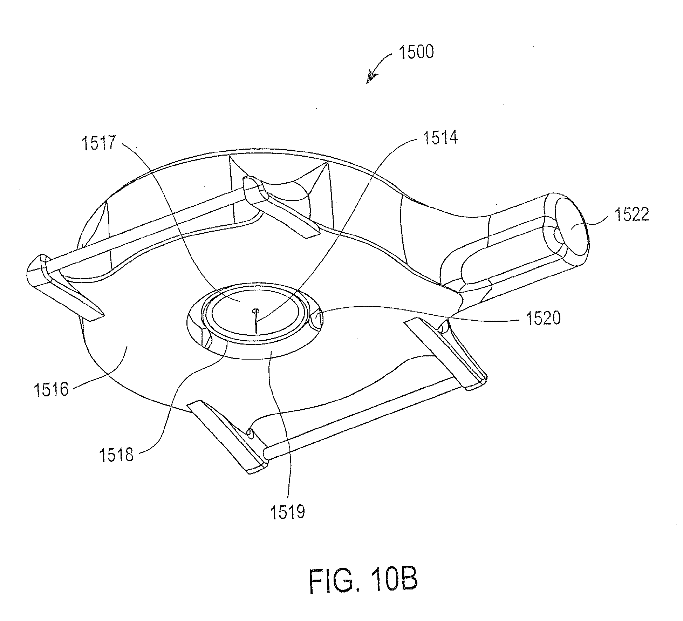

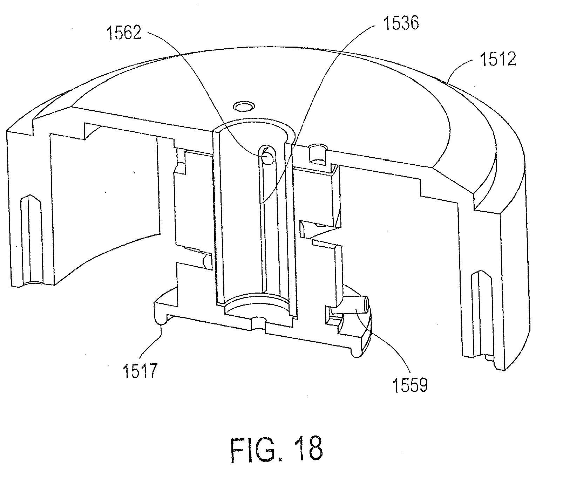

It is preferable that inner pressure ring 1517 and outer pressure ring 1518 not rotate when contacting the user's skin layer. The combination of possible pressure and rotation may cause bruising and discomfort to a user. Outer pressure ring 1518 further includes at least one linear groove 1534 on its outermost surface as shown in FIG. 17. Linear groove 1534 is positioned on the outermost surface of outer pressure ring 1518 which is parallel to the upward and downward linear movement of outer pressure ring 1518. Main housing 1509 has three tabs 1520 (see FIG. 10B) which are keyed to three linear grooves 1534 and prevent pressure ring 1518 from rotating. FIG. 18 shows a perspective cross-sectional view of upper housing 1512 mounted onto inner pressure ring 1517. Upper housing 1512 further includes two linear notches 1562 (only one linear notch is shown in FIG. 18). Similar to outer pressure ring 1518, linear notch 1562 is positioned parallel to the linear movement of inner pressure ring 1517. Inner pressure ring 1517 has two corresponding fixing pins 1562 which are fixedly mounted thereon. The two fixing pins 1562 are keyed to linear notch 1536 as shown in FIG. 18 and prevent inner pressure ring 1517 from rotating.

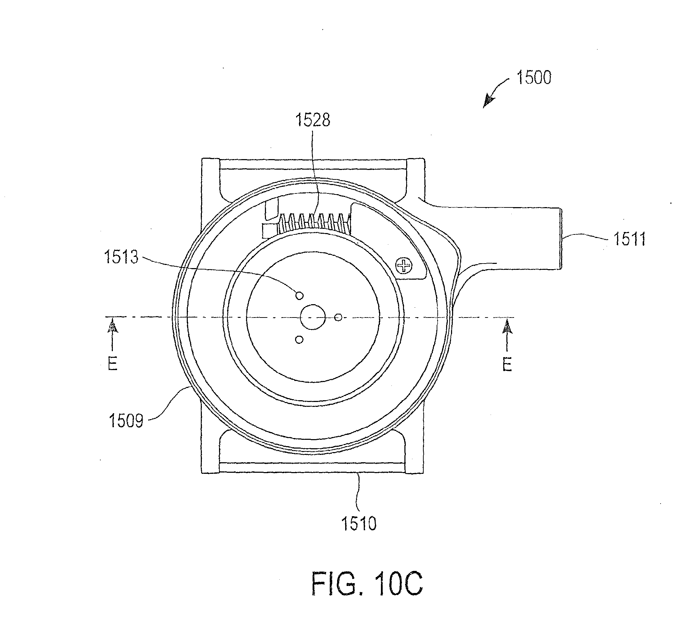

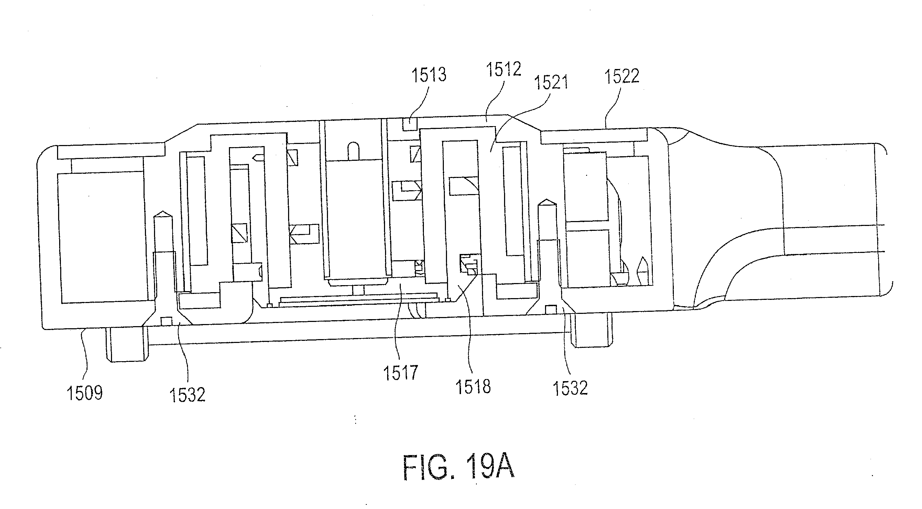

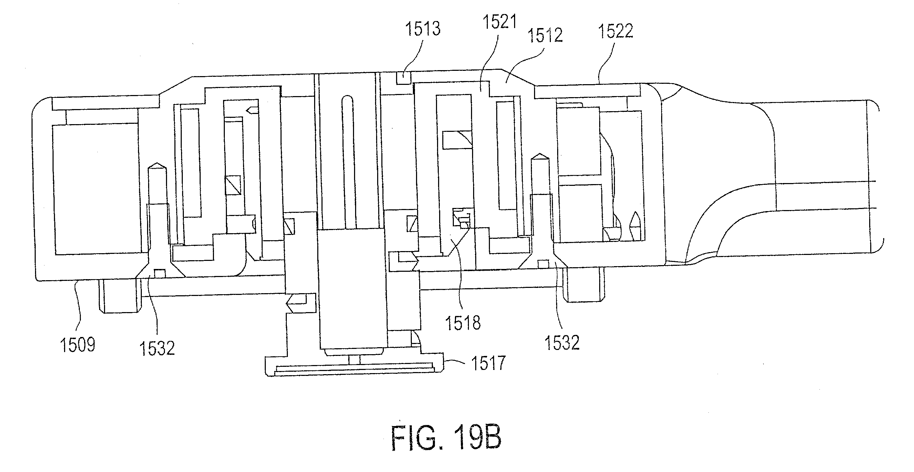

FIG. 19A is a cross-sectional side view of ISF extraction device 1500 along line E-E' as shown in FIG. 10C before lancing. ISF extraction device 1500 may be mounted to the body so that a glucose measurement cycle may be initiated. Next, inner pressure ring 1517 is lowered towards the user's skin layer as shown in FIG. 19B. This helps make the user's skin layer taut so that a penetration member may be launched.

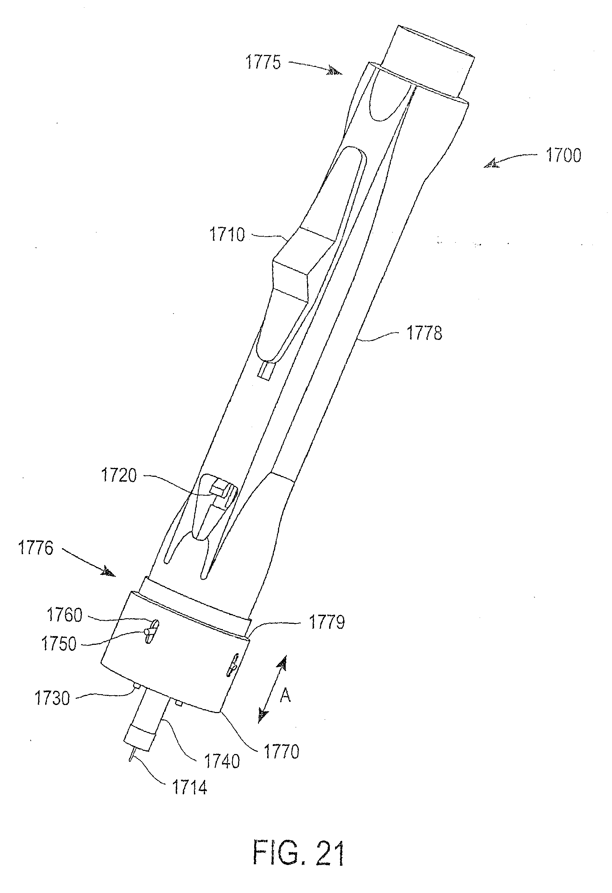

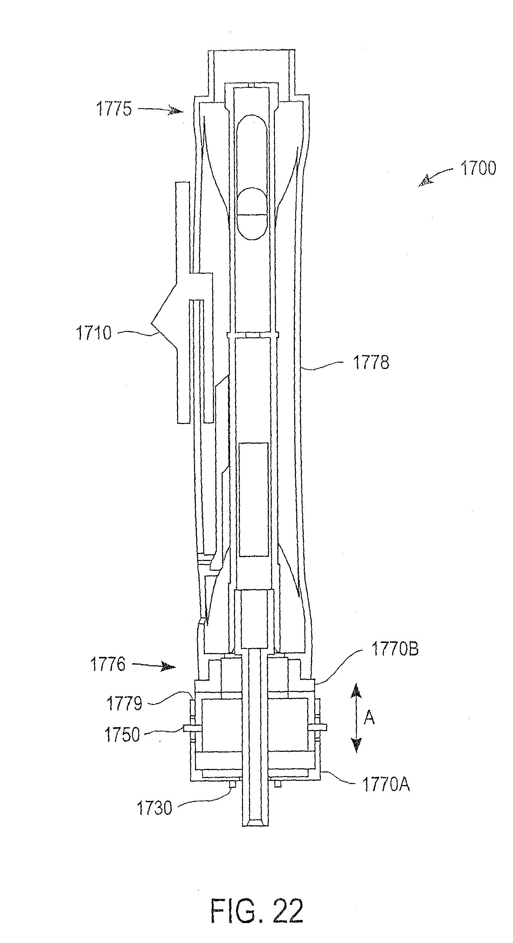

In an embodiment of the present invention, lancing mechanism 24 (see FIG. 2) may be implemented as an external lancing module 1700 which is shown in FIGs. 21 and 22. FIG. 21 shows a perspective view of external lancing module 1700 which includes a body 1778, a lever 1710, a switch 1720, an optional analysis module 1740, a cap 1770, a notch 1760, a pressure guiding peg 1750, a lance positioning peg 1730, and a penetration member 1714. External lancing module 1700 may have a proximal end 1775 and a distal end 1776 as shown in FIG. 21. External lancing module 1700 may be constructed using a modified One Touch® UltraSoft lancing device which is commercially available from LifeScan. The biasing elements were replaced so that they may be adapted for launching penetration member 1714 and the cap was replaced with a modified cap having a pressure feedback loop functionality. The mechanical structure of the OneTouch® UltraSoft lancing device is described in United State patents US 6045567, US6197040, and US6156051, all of which are hereby fully incorporated by reference herein. External lancing module 1700 may be mated with ISF extraction device 1500 using lance positioner holes 1513 and correspondingly located lance positioning pegs 1730. More specifically, external lancing module 1700 would mate with ISF extraction device 1500 while inner pressure ring 1517 is lowered towards the user's skin layer as shown in FIG. 19B. This allows inner pressure ring 1517 to apply pressure to the user's skin layer and holding it taut before being able to launch penetration member 1714 into the user's skin layer.

In a preferred embodiment of this invention, a user would manually apply an additional amount of downward force with external lancing module 1700 towards the user's skin layer after mating with ISF extraction device 1500. In many instances, it would be difficult for the user ascertain the appropriate amount of manual force to be directed with external lancing module 1700. Therefore, external lancing module 1700 further includes a pressure feedback loop that guides the user to apply an appropriate amount of force in a reproducible manner.

The pressure feed back loop functionality is achieved by using cap 1770, a biasing element (not shown) within cap 1770, notch 1760, and pressure guiding peg 1750. FIG. 22 shows a cross-sectional view of external lancing module 1700. Cap 1770 may be in the form of a two part assembly that includes a lower portion 1770A and an upper portion 1770B which are slidingly engaged with each other. Cap 1770 may have a hollow cylindrical shape and may be removably engaged to distal end 1776. Lower portion 1770A may have a hole that allows penetration member 1714 to pass therethrough after actuating switch 1720. Lower portion 1770A may further have a biasing element such as, for example, a spring (not shown) mounted within the hollow cylindrical shape. Lower portion 1770A may further have at least one notch 1760 which is adapted to correspond to presssure guiding peg 1750 which is integrated or attached to upper portion 1770B. Notch 1760 may have a rectangular shape that is designed to guide the movement of lower portion 1770A using presssure guiding pegs 1750 in an upward and downward motion parallel to double arrow A as shown in FIGs 21 and 22. The boundary of notch 1760 limits both the extension and retraction of lower portion 1770A relative to upper portion 1770B.

In a rested state, the biasing element is fully extended such that external lancing device 1700 is ready to mate with ISF extraction device 1500. Once external lancing device 1700 is mated with ISF extraction device 1500, a manual downward force is applied that causes the biasing element to compress such that lower portion 1770A slidingly retracts along upper portion 1770B. Once pressure guiding peg 1750 touches the boundary of notch 1760, the user should notice a significant increase in the amount of downward force needed to further push external lancing device 1700. It is this feedback of increased resistance that should prompt the user to stop applying the downward force. At this point in time, the user should actuate switch 1720 to launch penetrations member 1714 towards the user's skin layer. In an embodiment of this invention, the biasing member, which is in this case a spring, has a force constant of about 1 Newton to about 10 Newtons, and preferably about 5 Newtons. It is the force of the spring which guides the user to apply the appropriate amount of downward force before actuating external lancing device 1700. In an embodiment of this invention, cap 1770 may have four radially spaced apart notches 1760 and four corresponding pressure guiding pegs 1750 to help guide the user in applying the appropriate amount of force.

In an alternative embodiment of external lancing device 1700, a labeling graduation (not shown) may be displayed adjacent to notch 1760 allowing a user to apply an intermediate amount of downward pressure on external lancing device 1700. Pressure guiding peg 1750 may be used to line up with a targeted level located on the labeling graduation. The use of the labeling graduations will allow a user to reproducibly apply an intermediate level of downward force. Because there may be variability from one user to another user, it may be desirable to customize the magnitude of manual downward force to reduce the amount of pain and also increase the likelihood of collecting a sufficient amount of ISF.

In yet another alternative embodiment of external lancing device 1700, a labeling graduation (not shown) may be displayed on upper portion 1770B. An uppermost edge 1779 of lower portion 1770A may be used to line up with a targeted level located on the labeling graduation displayed on upper portion 1774B.

It should be noted that external lancing device 1700 should not be limited for use with a continuous glucose monitor. ISF extraction device 1700 may be adapted for the purpose of obtaining a blood sample from a user's skin layer. In such an embodiment, cap 1770 would press directly against a user's skin layer instead of against an ISF extraction device 1500. The pressure feedback control mechanism of external lancing device 1700 will provide a user with an increased control in determining the appropriate amount of downward pressure to apply for reducing pain and increasing the likelihood of obtaining a sufficient amount of blood. Once a sufficient amount of blood has been collected, it may be tested using a disposable single use test strip such as for example a One Touch® Ultra® glucose test strip which is commercially available from LifeScan (Milpitas, California, USA).

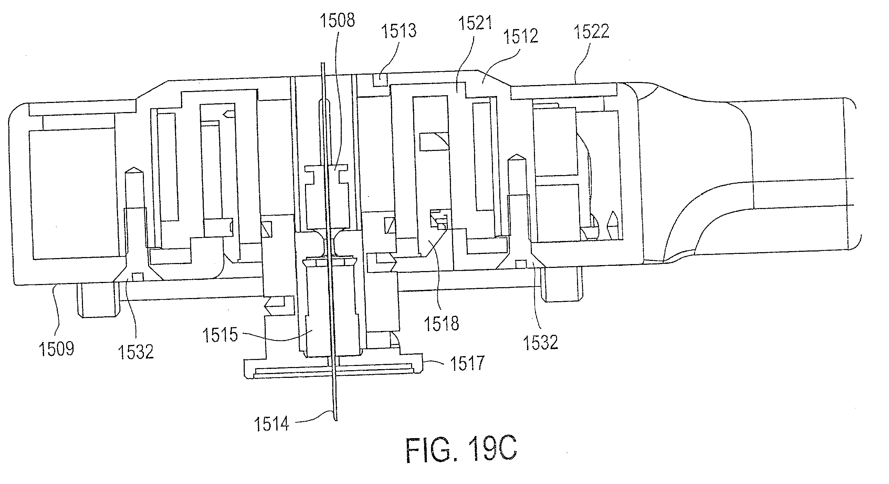

Lever 1710 may be cocked by an upward motion causing a spring to become pretensioned to an appropriate force and displacement. Switch 1720 may then be actuated causing penetration member 1714 to be launched into the user's skin layer so that penetration member 1714 may begin to collect ISF. Penetration member 1514 pierces the user's skin layer to a sufficient depth such that a physiological fluid such as ISF or blood flows into penetration member 1514 and transports the fluid to analysis module 1740, in this case a glucose a module. Lever 1710 may then be moved downward causing penetration member 1714 to be disengaged from lancing module 1700. In an embodiment of this invention, penetration member 1714 may be rigidly secured to upper housing 1512 using clamp 1508 which allows penetration member 1714 to remain in the user's skin layer for the duration of the measurement cycle. Clamp 1508 further includes 2 arms 1508A and 1508B which compress inwardly towards penetration member 1514 upon its insertion into inner pressure ring 1517 causing penetration member 1514 to be rigidly secured. FIG. 19C shows a cross-sectional view of ISF extraction device 1500 with penetration member 1714 already launched into the user's skin layer and secured to extraction device 1500.

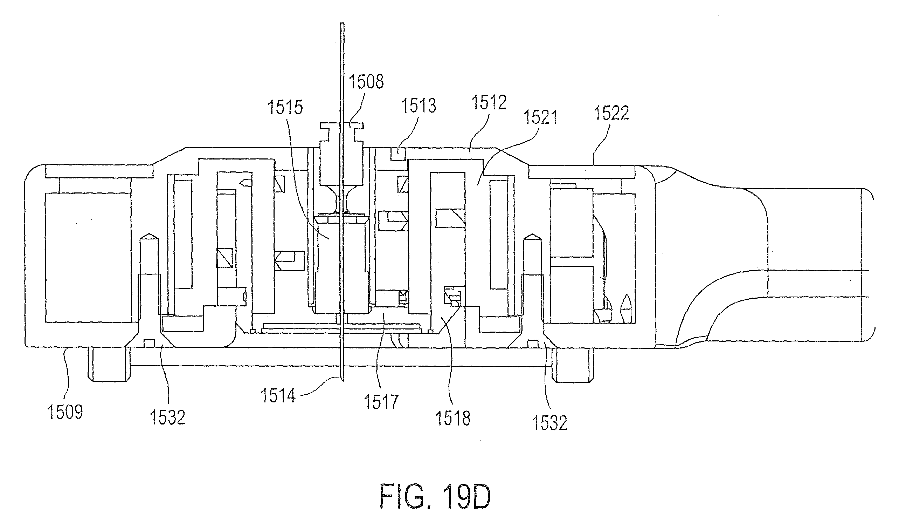

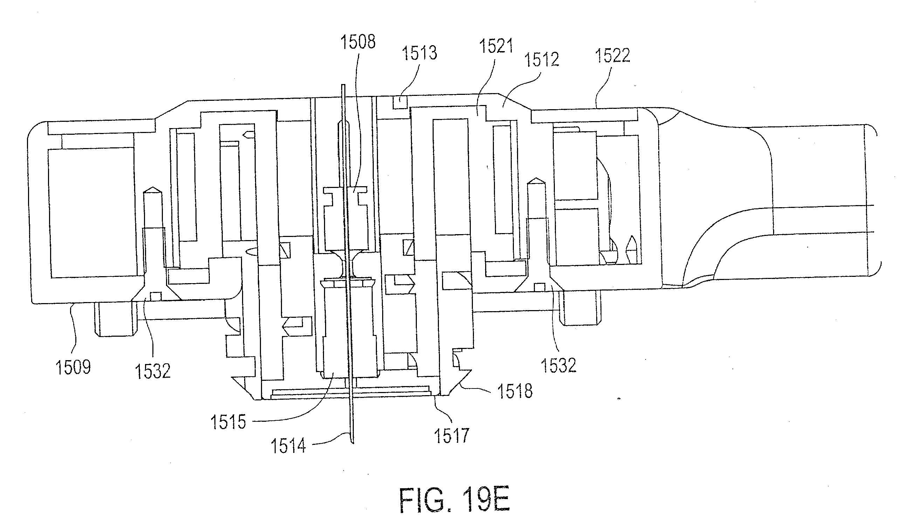

In an embodiment of this invention, inner pressure ring 1517 and outer pressure ring 1518 may be in one of three different states a) inner pressure ring 1517 is in the deployed state and outer pressure ring 1518 is in the retracted state as shown in FIG. 19C, b) both inner pressure ring 1517 and outer pressure ring 1518 are in the retracted state as shown in FIG. 19D, and c) both inner pressure ring 1517 and outer pressure ring 1518 are in the deployed state as shown in FIG. 19E. In an embodiment of the present invention, ISF extraction device 1500 may perform the following cycle by sequentially switching in order the following states - state a) for about 5 minutes, state c) for about 10 minutes, state b) for about 5 minutes; and state c) for about 10 minutes. It should be obvious to one skilled in the art that various sequences and durations of the three states may be implemented depending on the situation and/or individual.

It is preferable that ISF extraction device 1500 has a means to measure a linear displacement distance of inner pressure ring 1517. It is also preferable that ISF extraction device 1500 has a means to measure the amount of force that inner pressure ring 1517 or outer pressure ring 1518 is applying to the user's skin layer. A feedback force loop may be implemented allowing the linear displacement distance of inner pressure ring 1517 or outer pressure ring 1518 to be controlled based on the amount of pressure generated therefrom. This would enable programmed force thresholds to be achieved with either inner pressure ring 1517 and/or outer pressure ring 1518 for a prescribed duration of time. Under certain conditions, a customized force level may be tailored for a particular user so as to reduce person-to-person variability in extracting a targeted volume of ISF, rate of ISF expression, glucose lag, braising, and other desired attribute that have described herein.

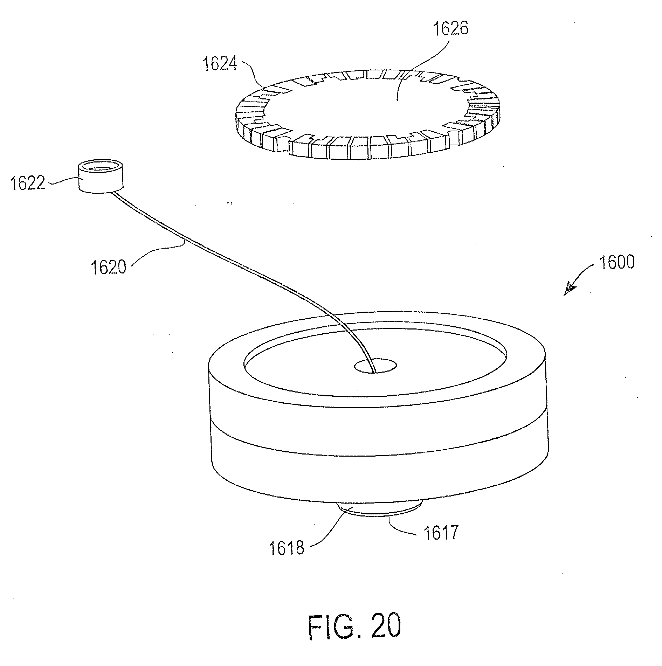

FIG. 20 is a simplified perspective view of an alternative embodiment of the invention in which an analysis module, in this case a glucose module, interfaces with another extraction device 1600. Extraction device 1600 includes an inner pressure ring 1617, an outer pressure ring 1618, a conduit 1620, and an adapter 1622. Inner pressure ring 1617 and outer pressure ring 1618 are the same as in extraction device 1500. In an alternative embodiment of this invention, ISF extraction device 1600 has an electronics portion therein which enable glucose to be measured electrochemically and also to control the movement of inner pressure ring 1617 and outer pressure ring 1618. Conduit 1620 may be made of a flexible material such as silicone to fluidly connect the penetration member to analysis module 1626 via adapter 1622. In an embodiment of this invention, the penetration member is rigidly attached to inner pressure ring 1617. Thus, the flexible material used for conduit 1620 allows for the oscillation of inner pressure ring 1617 between the retracted state and the deployed state while maintaining a fixed position for analysis module 1626. This may be preferable for the situation in which movement may affect ability of analysis module 1626 to accurately measure glucose. Analysis module 1626 includes a contact point 1624 for establishing electrical connection to the electronic portion of extraction device 1600. In an embodiment of this invention, contact points 1624 may be an electrically conductive co-injected material within analysis module 1626.

In an alternative embodiment of ISF extraction device 1600, analysis module 1626 may be rigidly connected to the penetration member without the use of conduit 1620. Therefore, if the penetration member is also rigidly connected to inner pressure ring 1617, analysis module 1626 will move with the penetration member between the deployed state and the retracted state. For situations in which movement does not affect the accuracy of analysis module 1626, it may be desirable that analysis module 1626 move during the measurement cycle so as to eliminate the use of conduit 1620 allowing the dead volume of ISF extraction device 1600 be reduced. It should also be noted that if analysis module 1626 is sufficiently large, then it would be undesirable for analysis module 1626 to move with inner pressure ring 1617 because the height of ISF extraction device would also become too large.

Inclusion of at least one pressure ring in extraction devices according to the present invention provides a number of benefits. First, oscillating the pressure ring(s) between a deployed and retracted state serves to mitigate (i.e., reduce) ISF glucose lag. Upon retraction of the pressure ring(s), pressure on the user's skin layer is released, and the user's body reacts by increasing blood perfusion to the target site. This phenomenon is known as reactive hyperemia and is hypothesized to be a mechanism by which ISF is beneficially replenished in the target site by oscillation of the pressure ring(s). Such a replenishment of ISF helps mitigating the lag between the ISF glucose and whole blood glucose values.

Another benefit of ISF extraction devices according to the present invention is that oscillation of the pressure ring(s) allows the skin under the pressure ring(s) to recover, thus reducing a user's pain, discomfort and the creation of persistent blemishes.

Moreover, extraction devices with a plurality of pressure rings (e.g., the embodiment of FIGs. 5-6 and 10-11) may be used with at least one pressure ring permanently deployed to facilitate ISF collection while the other pressure rings are oscillated between deployed and retracted states so that different areas of the user's skin layer are under pressure at any given time. Such combination of permanently deployed pressure ring(s) and oscillated pressure ring(s) further aids in reducing a user's pain/discomfort.

Still another benefit of ISF extraction devices according to the present embodiment is that the pressure ring(s) may be used to control the conditions under which a glucose measurement of an extracted ISF sample is conducted. For example, an electrochemical glucose sensor is more accurate and precise if the ISF sample flow rate past the glucose sensor is constant or static. The pressure ring(s) of ISF extraction devices according to the present invention may provide a controlled flow of the extracted ISF sample. For example, retraction of the pressure ring(s) may stop ISF sample flow for a time period of 0.1 seconds to 60 minutes to allow a glucose concentration measurement to be conducted. Once the glucose concentration measurement is complete, one or more of the pressure rings may be redeployed to continue ISF extraction. In this manner, a semi-continuous ISF sample extraction may be accomplished.

Once apprised of the present disclosure, one skilled in the art will recognize that ISF extraction devices according to the present invention may be employed in a variety of systems including, but not limited to, systems for the extraction of a bodily fluid sample and monitoring of an analyte therein, as described above. For example, the ISF extraction devices may be employed in a sample module of such systems.

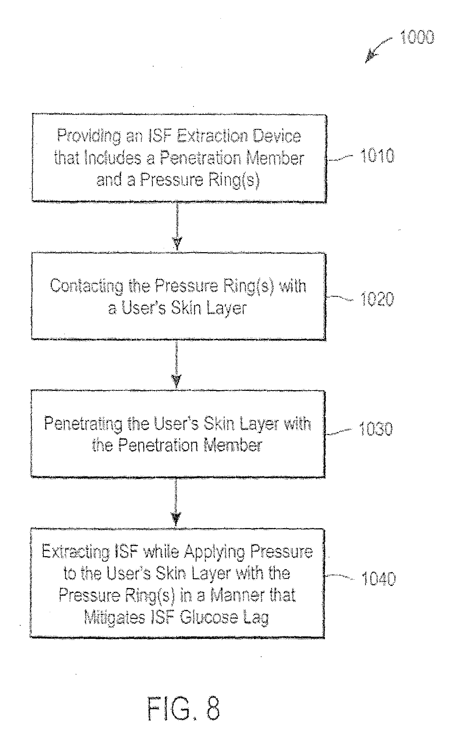

Referring to FIG. 8, a method 1000 for continuous collection of an ISF sample from a user's skin layer according to an exemplary embodiment of the present invention includes providing an ISF fluid extraction device, as set forth in step 1010. The ISF fluid extraction device that is provided includes a penetration member and at least one pressure ring (e.g., a single pressure ring or three concentric pressure rings). The penetration member and pressure ring(s) may be penetration members and pressure rings, as described above with respect to ISF extraction devices and systems according to the present invention.

Next, as set forth in step 1020, the pressure ring(s) is contacted with a user's skin layer in the vicinity of a target site (e.g., finger tip dermal tissue target site, a limb target site, an abdomen target site or other target site from which an ISF sample is to be extracted). The pressure ring may be contacted to the user's skin layer using any suitable techniques including, for example, those described above with respect to embodiments of systems and devices according to the present invention.

The target site of the user's skin layer is then penetrated by penetration member, as set forth in step 1030. Next, ISF is extracted from the user's skin layer by the penetration member while pressure is applied to the user's skin layer in an oscillating manner that mitigates an ISF lag of the extracted ISF, as set forth in step 1040. The various oscillating manners, by which pressure is applied, in methods according to the present invention have been described above with respect to FIGs. 1-7, and 9-19.

The following examples serve to illustrate beneficial aspects of various embodiments of devices, systems and methods according to the present invention.

### EXAMPLES

Example 1: Impact of an oscillating pressure ring on blood perfusion in an area within the oscillating pressure ring. Laser Doppler image perfusion data were collected at semi-regular intervals from a 0.25 square centimeter area approximately centered in the inside of a pressure ring attached to a subject's forearm. The pressure ring had an outside diameter of approximately 1.35 cm and a wall thickness of approximately 0.08 cm. Baseline data were collected prior to deploying the pressure ring against the subject's skin layer. The pressure ring was deployed against the skin layer for 10 minutes with a spring force of 0.5 lbs, retracted from the skin layer for 30 minutes, and then this cycle of deployment and retraction was repeated. The pressure ring was subsequently deployed against the skin layer for 5 hours, raised for 1 hour, and finally deployed against the skin for 10 minutes. The average perfusions in the 0.25 cm sq. measurement area are shown in the graph of FIG. 7.

As may be seen in the graph in FIG. 7, deployment of the pressure ring reduced blood perfusion in the area enclosed by the pressure ring (i.e., blood perfusion was reduced with the application of pressure), in comparison to the baseline blood perfusion. However, removing the pressure ring (i.e., releasing the pressure) not only reversed this effect, but actually increased perfusion beyond the baseline.

Example 2: Impact of an oscillating pressure ring on ISF glucose lag. A study was performed to determine the impact of blood flow on ISF glucose values during use of an oscillating pressure ring according to exemplary embodiments of the present invention. Twenty diabetic subjects underwent a procedure, in which baseline blood perfusion measurements were made on volar (palm) and dorsal portions of the subject's forearms. The subjects then participated in a test, in which finger blood samples, control ISF samples and treated ISF samples were collected at 15-minute intervals over a period of 3 to 6 hours. Control ISF samples were obtained from the subject's forearms without any skin layer manipulation and treated ISF samples were obtained by manipulating the subject's skin layer with an oscillating pressure ring. During the 3 to 6 hour testing period, blood glucose was influenced by ingestion of a microwave meal and diabetes medications including insulin and oral hypoglycemics such that most subjects experienced a rise and fall in blood glucose.

The treated ISF samples were created by applying approximately 150 pounds per square inch of pressure with a pressure ring with no sampling for 30 seconds, followed by a 5 minute waiting period to allow blood to perfuse into the sampling target site. Blood perfusion measurements were made with a Moor Laser Doppler Imager (Devon, UK) immediately prior to obtaining both control and treated ISF samples. Laser Doppler imaging was performed over a 2 square centimeter area centered on the ISF sampling target site.

Lag times in minutes and perfusion measurements are given in Table 1 for each subject.

**TABLE 1**

| Subject | control an blood units | treatment blood per units | treatment blood per ratio | control IS lag (min.) | treatment overal lag | overall lag mitigation |
|---|---|---|---|---|---|---|
| 8 | 97.1 | 212.9 | 2.19 | 30 | 10 | 20 |
| 9 | 65.3 | 170.3 | 2.61 | 21 | 5 | 16 |
| 10 | 84.0 | 187.6 | 2.23 | 26 | 4 | 22 |
| 11 | 50.2 | 117.3 | 2.34 | 22 | -5 | 27 |
| 12 | 68.4 | 223.5 | 3.27 | 12 | -2 | 14 |
| 13 | 95.4 | 295.2 | 3.09 | 30 | 15 | 15 |
| 14 | 62.0 | 150.3 | 2.42 | 47 | 12 | 35 |
| 15 | 51.7 | 92.8 | 1.80 | 50 | 10 | 40 |
| 16 | 80.0 | 80.9 | 1.01 | 41 | 24 | 17 |
| 17 | 64.6 | 107.9 | 1.67 | 46 | 12 | 34 |
| 18 | 101.2 | 244.4 | 2.41 | 50 | 11 | 39 |
| 19 | 86.2 | 142.4 | 1.65 | 27 | 16 | 11 |
| 20 | 114.8 | 256.9 | 2.24 | 42 | 16 | 26 |
| 21 | 118.6 | 198.3 | 1.67 | 13 | 5 | 8 |
| 22 | 73.2 | 156.2 | 2.13 | 25 | 8 | 17 |
| 23 | 114.7 | 278.2 | 2.43 | 30 | 8 | 22 |
| 24 | 94.4 | 253.6 | 2.69 | 15 | 8 | 7 |
| 25 | 161.2 | 482.0 | 2.99 | 8 | -2 | 10 |
| 26 | 58.7 | 151.7 | 2.59 | 42 | 9 | 33 |
| 27 | 114.6 | 363.3 | 3.17 | 29 | 8 | 21 |
| 28 | 56.3 | 117.0 | 2.08 | 31 | 10 | 21 |
| **mean:** | **86.3** | **203.9** | **2.32** | **30.3** | **8.7** | **21.7** |
| SD: | 28.1 | 97.2 | 0.6 | 12.8 | 6.6 | 9.9 |

The data in Table 1 show that ISF glucose lag was mitigated an average of 21.7 minutes, i.e., from a mean of 30.3 minutes (12.8 SD) to a mean of 8.7 minutes (6.6 SD) by use of the oscillating pressure ring. This lag mitigation was accomplished by the application and release of pressure to the subject's skin layer in a manner that caused an elevation of local blood perfusion in the ISF sampling areas by an average of 2.3 times (0.6 SD) relative to control sampling areas.

While preferred embodiments of the present invention have been shown and described herein, it will be obvious to those skilled in the art that such embodiments are provided by way of example only. Numerous variations, changes, and substitutions will now occur to those skilled in the art without departing from the invention.

It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A system for extracting bodily fluid from the skin, the system comprising:
a penetration member configured for penetrating a target site in the skin and accessing bodily fluid therein; and
means for applying pressure to the skin in an oscillating manner, the pressure application means extending substantially concentrically about the penetration member.

2. The system of claim 1 wherein the means for applying pressure comprises at least one ring concentrically positioned about the penetration member.

3. The system of claim 2 wherein the means for applying pressure further comprises a rotatable gear wheel, wherein the gear wheel is adapted to interact with the at least one pressure ring such that rotation of the gear wheel in a first direction imparts translational motion to the at lest one pressure ring progressively towards the user's skin layer allowing pressure to be applied to the user's skin layer and rotation of the gear wheel in a second direction, opposite to the first direction, imparts translational motion to the at least one pressure ring progressively away from the user's skin layer.

4. The system of claim 1, 2 or 3 further comprising an analysis module which is adapted to receive the accessed bodily fluid.

5. The system of any of the preceding claims wherein the system is configured to be worn on the body.

6. A method for extracting bodily fluid from the skin, the method comprising;
penetrating a target site in the skin and accessing bodily fluid therein; and
applying pressure to the skin in an oscillating manner substantially concentrically about the target site wherein extraction of fluid from the skin is facilitated.

7. The method of claim 6 wherein the pressure is applied at an oscillation frequency comprising applying a greater amount of pressure for a first time period and subsequently applying a lesser a mount of pressure for a second time period.

8. The system of claim 6 or 7 wherein the first time period is the same as the second time period.

9. The system of claim 6 or 7 wherein the first time period is different than the second time period.

10. The system of claim 9 wherein the first time period is longer than the second time period.

11. The system of claim 9 wherein the first time period is shorter than the second time period.

12. The system of any of the preceding claims wherein the applying the lesser amount of pressure comprises applying no pressure.

13. The system of any of the preceding claims wherein the pressure applied is in the range from about 0.1 to about 150 pounds per square inch to the skin.
